# EUROPEAN PATENT APPLICATION

(11) **EP 4 656 651 A1**
(43) Date of publication of application: **03.12.2025**
(21) Application number: 24775239.7
(22) Date of filing: 22.03.2024
(51) Int. Cl.: C07K 14/34, C12N 15/77, C12P 13/06, C12P 13/12, C12P 13/08

(54) **VARIANTS OF THREONINE/HOMOSERINE EFFLUX PROTEIN AND L-AMINO ACID PRODUCTION METHOD USING SAME**

(30) Priority: 23.03.2023 KR 20230038209
(71) Applicant: CJ CheilJedang Corporation, Seoul 04560 (KR)
(72) Inventor: EOM, Ga Eul, Seoul 04560 (KR); KIM, Moonjung, Seoul 04560 (KR); HUH, Lan, Seoul 04560 (KR); JANG, Yongjae, Seoul 04560 (KR); KIM, Kyungrim, Seoul 04560 (KR); SHIM, Jihyun, Seoul 04560 (KR)
(74) Representative: Meissner Bolte Partnerschaft mbB
(86) International application number: PCT/KR2024/003649
(87) International publication number: WO 2024/196193

(57) **Abstract**

Provided are a threonine/homoserine export protein variant, and a method of producing an L-amino acid using the same.

## Description

### [Technical Field]

The present disclosure relates to a threonine/homoserine export protein variant, and a method of producing an L-amino acid using the same.

### [Background Art]

L-homoserine, which is an intermediate in the methionine biosynthesis pathway and threonine biosynthesis pathway (WO2008/013432), is used as a precursor for the production of methionine and threonine. L-homoserine is synthesized by reduction from L-aspartate-4-semialdehyde by homoserine dehydrogenase (hom).

In order to produce L-amino acids such as L-homoserine and other useful substances, various studies have been conducted to develop microorganisms with high-efficiency production and fermentation process technologies. For example, target material-specific approaches are mainly used, such as increasing the expression of a gene encoding an enzyme involved in L-homoserine biosynthesis or deleting a gene unnecessary for the biosynthesis.

However, studies are still needed to effectively increase the production capacity of L-amino acids, comprising L-homoserine, in microorganisms.

### [Disclosure]

### [Technical Problem]

The present inventors found that a microorganism expressing a threonine/homoserine export protein variant of the present disclosure produces L-amino acids in high yields, thereby completing the present disclosure.

### [Technical Solution]

An aspect of the present disclosure provides a threonine/homoserine export protein variant, in which an amino acid corresponding to position 123 in an amino acid sequence of SEQ ID NO: 1 is substituted with a different amino acid.

In one specific embodiment, the different amino acid may be an amino acid selected from the group consisting of alanine, cysteine, aspartic acid, glutamic acid, phenylalanine, glycine, histidine, isoleucine, lysine, leucine, methionine, asparagine, proline, glutamine, arginine, serine, threonine, tryptophan, and tyrosine.

In another specific embodiment, the different amino acid may be an amino acid selected from the group consisting of alanine, cysteine, aspartic acid, phenylalanine, glycine, histidine, isoleucine, leucine, methionine, asparagine, glutamine, serine, threonine, tryptophan, and tyrosine.

Another aspect of the present disclosure provides a polynucleotide encoding the threonine/homoserine export protein variant of the present disclosure.

Still another aspect of the present disclosure provides a microorganism comprising any one or more selected from the threonine/homoserine export protein variant of the present disclosure; the polynucleotide encoding the same; or a vector comprising the polynucleotide.

In one specific embodiment, the microorganism may have an increased L-amino acid-producing ability compared to a microorganism comprising a wild-type threonine/homoserine export protein having an amino acid sequence of SEQ ID NO: 1 or a polynucleotide encoding the same.

In another specific embodiment, the L-amino acid may be any one or more selected from the group consisting of L-homoserine, O-acetyl-L-homoserine, O-succinyl-L-homoserine, L-methionine, and L-threonine.

With regard to the microorganism according to any one of the aforementioned specific embodiments, the microorganism may be a microorganism of the genus *Corynebacterium.*

With regard to the microorganism according to any one of the aforementioned specific embodiments, the microorganism of the genus *Corynebacterium* may be *Corynebacterium glutamicum.*

Still another aspect of the present disclosure provides a method of producing an L-amino acid, the method comprising the step of culturing, in a medium, the microorganism comprising any one or more selected from the threonine/homoserine export protein variant of the present disclosure; the polynucleotide encoding the same; or a vector comprising the polynucleotide.

Still another aspect of the present disclosure provides a composition for producing an L-amino acid, the composition comprising the microorganism comprising any one or more selected from the threonine/homoserine export protein variant of the present disclosure; the polynucleotide encoding the same; or a vector comprising the polynucleotide, a culture of the microorganism, a fermentation product of the microorganism, or a combination of two or more thereof.

### [Advantageous Effects]

A microorganism expressing a threonine/homoserine export protein variant of the present disclosure is able to produce an L-amino acid in high yield, thereby being usefully applied to the production of the L-amino acid.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definition

As used in the specification and appended claims of the present disclosure, the singular forms ("a", "an", and "the") comprise plural referents unless the context clearly states otherwise. Unless the context indicates otherwise, singular terms shall comprise the plural, and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third," "i), ii), iii)..." or "(a), (b), (c), (d)..." are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

### Polypeptide

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, an amino acid sequence exhibiting activity may be referred to as an "enzyme". In the present disclosure, unless indicated otherwise, amino acid sequences are described in an N-terminal to C-terminal direction.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of post-translational modification comprise N- or C-terminal modifications, glycosylation, phosphorylation, leader sequence removal etc., but are not limited thereto.

With respect to amino acid sequences in the present disclosure, although it is described as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted, or added may be within the scope of the present disclosure as long as it has identical or corresponding activity to that of the protein consisting of the amino acid sequence of the corresponding sequence number. For example, as long as a protein has identical or corresponding activity to that of the modified protein, it does not exclude the addition of a sequence that does not change the function of the protein before or after the amino acid sequence, a naturally occurring mutation, a silent mutation thereof, or a conservative substitution, and it is apparent that even though the protein has such sequence additions or mutations, it falls within the scope of the present disclosure.

For example, it may be a case where a protein has the addition of a sequence that does not change the function of the polypeptide of the present disclosure at the N-terminus, C-terminus, and/or inside of the amino acid sequence, a naturally occurring mutation, a silent mutation, or a conservative substitution. For example, the polypeptide may be conjugated to an N-terminal signal (or leader) sequence that is co-translationally or post-translationally involved in the protein transfer. Further, the polypeptide may be conjugated to another sequence or linker so as to enable identification, purification, or synthesis of the polypeptide.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids comprise arginine, lysine, and histidine; negatively charged (acidic) amino acids comprise glutamic acid and aspartic acid; amino acids having a nonpolar side chain (nonpolar amino acids) comprise glycine, alanine, valine, leucine, isoleucine, methionine, phenylalanine, tryptophan, and proline; amino acids having a polar or hydrophilic side chain (polar amino acids) comprise serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, when the 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto. Usually, conservative substitution may have little to no effect on the activity of polypeptides.

### Polynucleotide

As used herein, the term "gene" means a polynucleotide that encodes a polypeptide and a polynucleotide comprising the upstream and downstream regions of the coding region. In some specific embodiments, a gene may have a sequence (intron) inserted between each coding region (exon).

As used herein, the term "polynucleotide, nucleic acid, or nucleic acid molecule" refers to a DNA (e.g., cDNA or genomic DNA) or RNA (e.g., mRNA) strand of a certain length or longer as a polymer of nucleotides in which nucleotide monomers are connected in a long chain by covalent bonds.

### Homology, Identity

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program to be used may be used together. Substantially, homologous or identical sequences may generally hybridize under moderately or highly stringent conditions to the entirety of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al. (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO et al.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may comprise: (1) unitary matrices (comprising a value 1 for identity and a value 0 for non-identity), PAM Matrix (see those described by Schwartz and Dayhoff, eds., Atlas Of Protein Sequence And Structure, National Biomedical Research Foundation (1978)), and the weighted comparison matrix of Gribskov et al(1986) Nucl. Acids Res. 14: 6745 (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

Further, whether any two polynucleotide or polypeptide sequences have homology, similarity, or identity may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but is not limited thereto.

As used herein, the term "stringent conditions" means conditions that enable specific hybridization between polynucleotides. These conditions are specifically described in documents (see Sambrook et al., supra, 9.50-9.51, 11.7-11.8). Examples thereof comprise conditions in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or conditions in which washing is performed once, specifically twice to three times at a salt concentration and temperature equivalent to 60°C, 1×SSC, 0.1% SDS, specifically 60°C, 0.1×SSC, 0.1% SDS, more specifically, 68°C, 0.1×SSC, 0.1% SDS, which are washing conditions for common Southern hybridization.

The hybridization requires that two nucleotides have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar base sequences as well as isolated nucleic acid fragments that are complementary to the entire sequence.

For example, polynucleotides having homology or identity to the polynucleotide of the present disclosure may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency to hybridize the polynucleotide depends on the length and degree of complementarity of the polynucleotide, and the variables are well known in the art (e.g., J. Sambrook et al., supra).

### Nucleic Acid Construct, Vector, Transformation

As used herein, the term "nucleic acid construct" refers to a single- or doublestranded nucleic acid molecule, which comprises one or more regulatory sequences, and which is artificially synthesized, or engineered to comprise a specific sequence in a manner that does not exist in nature, or isolated from nature.

As used herein, the term "vector" means a DNA construct comprising a nucleotide sequence of a polynucleotide encoding a target polypeptide which is operably linked to a suitable expression regulatory region (or expression regulatory sequence) so that the target polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicate or function independently of the host genome, or may be integrated into the genome itself.

The vector used in the present disclosure is not particularly limited, but any vector known in the art may be used. Examples of commonly used vectors may comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, a pDZ, pDC, pACYC177, pACYC184, pCL, pCL1920, pSHK130, pDCM2, pECCG117, pUC19, pBR322, pMW118, pCC1BAC vector, etc. may be used.

For example, a polynucleotide encoding a target polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for checking the chromosome insertion. The selection marker is for selecting the cells transformed with vectors, i.e., for checking the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or microorganism so that the polypeptide encoded by the polynucleotide is expressed in the host cell. As long as the transformed polynucleotide may be expressed in the host cell, it may be integrated into and located in the chromosome of the host cell or exist extrachromosomally, or it may be in any of these cases. Further, the polynucleotide comprises DNA or RNA encoding the target polypeptide. The polynucleotide may be introduced in any form as long as it may be introduced into a host cell and expressed therein. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

As used herein, the term "operably linked" refers to a configuration of placing a regulatory sequence in an appropriate position to direct expression of a coding sequence. Thus, the term "operably linked" comprises an attachment or linking between a regulatory region of a functional domain having a known or desired activity such as a promoter, a terminator, a signal sequence, or an enhancer, and a target (gene or polypeptide) such that the expression, secretion, or function of the target (gene or polypeptide) may be regulated in accordance with the known or desired activity. For example, the term means that the polynucleotide sequence is functionally linked to a promoter sequence that initiates and mediates transcription of the polynucleotide encoding the polypeptide of the present disclosure.

As used herein, the term "expression" comprises any step involved in the production of a polypeptide, e.g., transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression thereof.

As used herein, the term "regulatory sequence" refers to a polynucleotide sequence required for the expression of a coding sequence. Each regulatory sequence may be native (derived from the same origin) or foreign (derived from different genes) to the coding sequence. Examples of the regulatory sequence may comprise a leader sequence, a polyadenylation sequence, a propeptide sequence, a promoter, a signal peptide sequence, an operator sequence, a ribosome binding site-encoding sequence, and a sequence terminating transcription and translation. A minimum unit of the regulatory sequence may comprise a promoter and a sequence terminating transcription and translation.

With regard to a cell, polynucleotide, polypeptide, or vector, the term "recombinant" used herein refers to a cell, polynucleotide, polypeptide, or vector modified by the introduction of a heterologous nucleic acid or polypeptide or alteration of a native polynucleotide or polypeptide, or a cell derived from the modified cell. Thus, for example, recombinant cells may express genes that are not found within the native (non-recombinant) form of the cells, or may express native genes that are otherwise abnormally expressed, underexpressed or not expressed at all.

### Microorganism

As used herein, the term "microorganism (or strain)" comprises all wild-type microorganisms, or microorganisms in which genetic modification naturally or artificially occurs, and it may be a microorganism in which a specific mechanism is weakened or enhanced due to insertion of a foreign gene or an activity enhancement or inactivation of an endogenous gene, and it may be a microorganism comprising genetic modification for the production of a desired polypeptide, protein, or product. As used herein, the "microorganism" and "strain" have the same meaning and may be used interchangeably without limitation.

For example, the microorganism of the present disclosure may be a microorganism comprising the threonine/homoserine export protein variant or the polynucleotide encoding the same; or a microorganism (e.g., recombinant strain) genetically modified to comprise the threonine/homoserine export protein variant or the polynucleotide encoding the same.

The strain of the present disclosure may be a strain naturally having an L-amino acid-producing ability or a microorganism prepared by providing the L-amino acid-producing ability for a strain without the L-amino acid-producing ability, for example, a microorganism in which the L-amino acid-producing ability is increased by introducing the threonine/homoserine export protein variant of the present disclosure or the polynucleotide encoding the same, but is not limited thereto.

The strain of the present disclosure may be a microorganism with increased L-amino acid-producing ability, as compared to a parent strain without the variant of the present disclosure or a wild-type strain of the genus *Mycobacterium.* The microorganism may be a microorganism in which the L-amino acid-producing ability is improved by introducing the threonine/homoserine export protein variant of the present disclosure having the increased threonine/homoserine export activity, as compared to the wild-type threonine/homoserine export protein, or the polynucleotide encoding the same. Specifically, the strain of the present disclosure may have the increased L-amino acid-producing ability, as compared to a microorganism of the genus *Corynebacterium* comprising the wild-type threonine/homoserine export protein having the amino acid sequence of SEQ ID NO: 1 or a polynucleotide encoding the same.

As used herein, the term "microorganism having the L-amino acid-producing ability" refers to a prokaryotic or eukaryotic microbial strain capable of producing an L-amino acid within a living organism, and may comprise both a microorganism prepared by providing the L-amino acid-producing ability for a parent strain without the L-amino acid-producing ability, or a microorganism inherently having the L-amino acid-producing ability. The L-amino acid-producing ability may be conferred or enhanced by species improvement.

As used herein, the term "unmodified microorganism" does not exclude strains comprising a mutation that may occur naturally in microorganisms, and may refer to a wild-type strain or a natural strain itself or may be a strain before the trait is changed by genetic variation due to natural or artificial factors. The "unmodified microorganism" may be used interchangeably with a "strain before being modified", "microorganism before being modified", "unvaried strain", "unmodified strain", "unvaried microorganism", "parent strain before being modified", "wild-type microorganism", "reference microorganism", or "standard microorganism". Further, as used herein, the term "threonine/homoserine export protein-unmodified microorganism" may refer to a strain into which the threonine/homoserine export protein variant described herein is not introduced or has not yet been introduced. The threonine/homoserine export protein-unmodified microorganism of the present disclosure does not exclude a strain comprising modification of proteins or genes other than the modification of the threonine/homoserine export protein or the polynucleotide encoding the same. Further, in the present disclosure, the unmodified microorganism may be a microorganism comprising the amino acid sequence consisting of SEQ ID NO: 1 or a polynucleotide consisting of SEQ ID NO: 2, but is not limited thereto.

### Increase of Polypeptide Activity

As used herein, the term "increase" in the activity of the polypeptide means that the activity of the polypeptide is enhanced compared with the endogenous activity. The increase may be used interchangeably with the terms activation, up-regulation, overexpression, enhancement, etc.

The increase may comprise both cases in which an activity not originally possessed is exhibited, or the activity is enhanced, compared to the endogenous activity or the activity before modification.

For example, "the activity not originally possessed is exhibited" may be "introduction of a protein", but is not limited thereto. The introduction of the protein means that a gene not originally possessed by a microorganism is expressed within the microorganism to exhibit the activity of a specific protein or to exhibit the increased or improved activity compared to the endogenous activity of the corresponding protein or the activity before modification. For example, a polynucleotide encoding a specific protein may be introduced into a chromosome in a microorganism, or a vector comprising a polynucleotide encoding the specific protein may be introduced into the microorganism, thereby exhibiting its activity.

The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification".

The fact that the activity of a polypeptide is increased compared to the endogenous activity means that the activity of the polypeptide is improved compared to the activity and/or concentration (expression level) of the specific polypeptide originally possessed by a parent strain before the trait is changed or an unmodified microorganism.

For example, the increase indicates that the activity of the corresponding protein originally absent appears, or its activity or concentration is generally increased to about 1%, about 10%, about 25%, about 50%, about 75%, about 100%, about 150%, about 200%, about 300%, about 400% or about 500%, up to about 1000% or about 2000% or more, based on the activity or concentration in the wild-type protein or the initial microbial strain, but is not limited thereto.

The increase of the activity of the polypeptide may be achieved through the introduction of a foreign polypeptide or the increase of the activity of the endogenous polypeptide. The increase of the activity of the polypeptide may be confirmed by an increase in the degree of activity and the expression level of the corresponding polypeptide or an increase in the amount of the product released from the corresponding polypeptide.

For the increase of the activity of the polypeptide, various methods well known in the art may be applied, and the method is not limited as long as the activity of the target polypeptide may be increased compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the polypeptide of the present disclosure may be:
1) increase in the intracellular copy number of the polynucleotide encoding the polypeptide;
2) modification of a gene expression regulatory region on a chromosome encoding the polypeptide (e.g., occurrence of variations in the expression regulatory region, replacement with a sequence having stronger activity, or insertion of a sequence having stronger activity);
3) modification of a nucleotide sequence encoding a start codon or 5'-UTR region of the gene transcript encoding the polypeptide;
4) modification of the amino acid sequence of the polypeptide to increase the activity of the polypeptide;
5) modification of the polynucleotide sequence encoding the polypeptide to increase the activity of the polypeptide (e.g., modification of the polynucleotide sequence of the polypeptide gene to encode the polypeptide that has been modified to increase the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

For example, 1) the increase in the intracellular copy number of the polynucleotide encoding the polypeptide may be achieved by the introduction, into a host cell, of a vector to which the polynucleotide encoding the corresponding polypeptide is operably linked and which is able to replicate and function independently of the host. Alternatively, the increase may be achieved by the introduction of one or two or more copies of the polynucleotide encoding the corresponding polypeptide into the chromosome in a host cell. The introduction into the chromosome may be performed by the introduction, into a host cell, of a vector capable of inserting the polynucleotide into the chromosome in the host cell, but is not limited thereto. The vector is as described above.

2) The replacement of a gene expression regulatory region (expression regulatory sequence) on the chromosome encoding the polypeptide with a sequence with strong activity may be, for example, the occurrence of variations on the sequence through deletion, insertion, non-conservative or conservative substitution, or a combination thereof, or the replacement with a sequence having stronger activity, to further enhance the activity of the expression regulatory region. The expression regulatory region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.

Examples of the known stronger promoters may comprise cj1 to cj7 promoters (US Patent No. 7662943 B2), lac promoter, trp promoter, trc promoter, tac promoter, lambda phage PR promoter, PL promoter, tet promoter, gapA promoter, rhtB promoter, SPL7 promoter, SPL13(sm3) promoter (US Patent No. 10584338 B2), O2 promoter (US Patent No. 10273491 B2), tkt promoter, yccA promoter, etc., but are not limited thereto.

3) The modification of a nucleotide sequence encoding a start codon or 5'-UTR region of the gene encoding the polypeptide may be, for example, replacing with another start codon having a higher polypeptide expression rate than the endogenous start codon, but is not limited thereto.

4) and 5) The modification of the amino acid sequence or polynucleotide sequence may be the occurrence of variations on the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence improved to have stronger activity or an amino acid sequence or polynucleotide sequence improved to have increased activity, in order to increase activity of the polypeptide, but is not limited thereto. Specifically, the replacement may be performed by inserting the polynucleotide into the chromosome by homologous recombination, but is not limited thereto. The vector used herein may further comprise a selection marker for checking the chromosome insertion. The selection marker is as described above.

6) The introduction of a foreign polynucleotide exhibiting the activity of the polypeptide may be the introduction, into a host cell, of a foreign polynucleotide encoding a polypeptide exhibiting identical/similar activity to the polypeptide. The foreign polynucleotide is not limited to the origin or sequence thereof as long as it exhibits identical/similar activity to the polypeptide. The method used in the introduction may be performed by any known transformation method appropriately selected by a person skilled in the art, and through the expression of the introduced polynucleotide in the host cell, the polypeptide is produced and the activity thereof may be increased.

7) The codon optimization of the polynucleotide encoding the polypeptide may be the codon optimization of an endogenous polynucleotide to increase transcription or translation thereof in a host cell, or the codon optimization of a foreign polynucleotide to allow optimized transcription or translation thereof in a host cell.

8) The modification or chemical modification of an exposed site selected by analysis of a tertiary structure of the polypeptide may be, for example, the modification or chemical modification of an exposed site to be modified or chemically modified by comparing sequence information of a polypeptide to be analyzed with a database that stores sequence information of known proteins, determining a template protein candidate according to similarity of the sequences, and identifying the structure based thereon.

Such an increase of the activity of the polypeptide may mean that the activity or concentration of the corresponding polypeptide is increased relative to the activity or concentration of the polypeptide expressed in the wild-type or the microbial strain before modification, or that the amount of a product produced from the corresponding polypeptide is increased, but is not limited thereto.

The modification of a part or the entirety of the polynucleotide in the microorganism of the present disclosure may be induced by (a) homologous recombination using a vector for chromosomal insertion in a microorganism or genome editing using engineered nuclease (e.g., CRISPR-Cas9), and/or (b) treatment with light, such as ultraviolet light and radiation, and/or chemicals, but is not limited thereto. The method of modifying a part or the entirety of the gene may comprise a method by DNA recombinant technology. For example, a nucleotide sequence or vector comprising a nucleotide sequence homologous to a target gene may be introduced into the microorganism to bring about homologous recombination, resulting in deletion in a part or the entirety of the gene. The nucleotide sequence or vector to be introduced may comprise a dominant selection marker, but is not limited thereto.

### Culturing

As used herein, the term "culturing" refers to growing the strain of the present disclosure under appropriately adjusted environmental conditions. The culture procedure of the present disclosure may be performed according to appropriate media or culture conditions known in the art. Such a culture procedure may be easily adjusted according to the selected strain by a person skilled in the art. Specifically, the culturing may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the strain of the present disclosure, any medium that is used for usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, while controlling the temperature, pH, etc. For example, a culture medium for the strains of the genus *Corynebacterium* may be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or decomposition products thereof, etc. may be used. These nitrogen sources may be used alone or in a combination of two or more thereof, but are not limited thereto.

The phosphate sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, sulfuric acid, etc., to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or non-aerobic state, but is not limited thereto.

In the culture of the present disclosure, the culture temperature may be maintained at 20°C to 45°C, specifically, at 25°C to 40°C, and the culture may be performed for about 10 hours to 160 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the specific microorganism to be separated by filtration, etc. is substantially excluded, which does not mean that the microorganism is completely excluded from the filtrate.). The formulation of the culture is not limited, but it may be, for example, a liquid, an emulsion, or a solid.

As used herein, the term "fermentation" refers to a process whereby microorganisms break down organic materials using their own enzymes, excluding putrefaction. Fermentation and putrefaction proceed by similar processes. However, when useful substances are produced as a result of decomposition, it is called fermentation, and when bad smells or harmful substances are produced, it is called putrefaction.

In the present disclosure, the method of obtaining the fermentation product from the strain is not particularly limited, and the fermentation product may be obtained according to a method commonly used in the art or similar art.

As used herein, the term "fermentation product" comprises not only a fermented material itself, but also all types of materials comprising the fermentation product generated from the strain, such as a culture medium of a strain in which the strain and the culture coexist, a fermentation product obtained by filtering the strain from the culture medium, a fermentation product obtained by sterilizing the strain from the culture medium and filtering the same, an extract obtained by extracting the fermentation product or the culture medium comprising the same, a dilution obtained by diluting the fermentation product or the extract thereof, a concentrate, a dry product obtained by drying the fermentation product or the extract thereof, a lysate obtained by collecting and disrupting cells of the strain, etc.

### Detailed Description of the Present Disclosure

Hereinafter, specific embodiments of the present disclosure will be described in more detail as follows.

An aspect of the present disclosure provides a threonine/homoserine export protein variant, in which an amino acid corresponding to position 123 in an amino acid sequence of SEQ ID NO: 1 is substituted with a different amino acid.

As used herein, the term "threonine/homoserine export protein variant" refers to any polypeptide having threonine and homoserine efflux activity or a variant comprising a substitution of the amino acid corresponding to position 123 from the N-terminus of SEQ ID NO: 1 with a different amino acid in the threonine/homoserine export protein. The "threonine/homoserine export protein variant" may also be referred to as "modified threonine/homoserine export protein", "variant RhtA", "RhtA variant", etc.

The threonine/homoserine export protein of the present disclosure, which is a subject of the introduction of the mutation, may also be referred to as "RhtA protein" or "RhtA", and may be a protein having threonine and homoserine efflux activity. Specifically, the protein may comprise the amino acid sequence of SEQ ID NO: 1 and may have the threonine and homoserine efflux activity, but is not limited thereto. Addition of a meaningless sequence upstream or downstream of the amino acid sequence of SEQ ID NO: 1, a naturally occurring mutation, or a silent mutation thereof is not excluded, and as long as a protein has activity identical or corresponding to that of the protein comprising the amino acid sequence of SEQ ID NO: 1, it may belong to the protein which is a subject of the introduction of the mutation of the present disclosure. For example, the protein which is a subject of the introduction of the mutation of the present disclosure may be a protein consisting of the amino acid sequence of SEQ ID NO: 1 or an amino acid sequence having 60%, 65%, 70%, 75%, 80%, 85%, 90%, 95%, 96%, 97%, 98%, or 99% or more homology or identity thereto. Further, it is obvious that any protein having an amino acid sequence with deletion, modification, substitution, or addition in part of the sequence may also fall within the scope of the protein which is a subject of the introduction of the mutation of the present disclosure, as long as it is an amino acid sequence having such homology or identity and exhibiting the efficacy corresponding to that of the protein.

In the amino acid sequence of the threonine/homoserine export protein before modification, which is the subject of the mutation in the present disclosure, i.e., in a sequence which may be a parent sequence, the amino acid before modification, corresponding to position 123 of SEQ ID NO: 1, may be valine (V).

With regard to the threonine/homoserine export protein variant of the present disclosure, the amino acid corresponding to position 123 in the amino acid sequence of SEQ ID NO: 1 may be substituted with an amino acid other than the amino acid before substitution.

For example, with regard to the threonine/homoserine export protein variant, the amino acid corresponding to position 123 in the amino acid sequence of SEQ ID NO: 1 may be substituted with an amino acid other than valine which is the amino acid before substitution.

For another example, with regard to the threonine/homoserine export protein variant, the amino acid corresponding to position 123 in the amino acid sequence of SEQ ID NO: 1 may be substituted with an amino acid selected from the group consisting of alanine, cysteine, aspartic acid, glutamic acid, phenylalanine, glycine, histidine, isoleucine, lysine, leucine, methionine, asparagine, proline, glutamine, arginine, serine, threonine, tryptophan, and tyrosine. Specifically, with regard to the threonine/homoserine export protein variant, the amino acid corresponding to position 123 in the amino acid sequence of SEQ ID NO: 1 may be substituted with an amino acid selected from the group consisting of alanine, cysteine, aspartic acid, phenylalanine, glycine, histidine, isoleucine, leucine, methionine, asparagine, glutamine, serine, threonine, tryptophan, and tyrosine.

For still another example, the threonine/homoserine export protein variant of the present disclosure may have, comprise, or consist of an amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25 or SEQ ID NO: 27 or SEQ ID NO: 29 or SEQ ID NO: 31 or SEQ ID NO: 33 or SEQ ID NO: 35 or SEQ ID NO: 37 or SEQ ID NO: 39, or may essentially consist of the amino acid sequence.

With regard to the threonine/homoserine export protein variant of the present disclosure, the amino acid corresponding to position 123 based on the amino acid sequence of SEQ ID NO: 1 may be an amino acid other than valine, for example, an amino acid selected from the group consisting of alanine, cysteine, aspartic acid, phenylalanine, glycine, histidine, isoleucine, leucine, methionine, asparagine, glutamine, serine, threonine, tryptophan, and tyrosine, and it may comprise an amino acid sequence having at least 60%, 65%, 70%, 75%, 80%, 85%, 90%, 95%, 96%, 97%, 98%, 99%, 99.1%, 99.2%, or 99.3% or more homology or identity to the amino acid sequence represented by SEQ ID NO: 1. It is also obvious that, as long as an amino acid sequence has such homology or identity and exhibits the efficacy corresponding to that of the threonine/homoserine export protein variant of the present disclosure, a threonine/homoserine export protein variant having an amino acid sequence with deletion, modification, substitution, conservative substitution, or addition in part of the sequence may also fall within the scope of the present disclosure.

For example, there are cases of having addition or deletion of a sequence that does not alter the function of the threonine/homoserine export protein variant of the present disclosure at the N-terminus, C-terminus, and/or within the amino acid sequence, a naturally occurring mutation, a silent mutation, or a conservative substitution.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of the residue. Usually, conservative substitution may have little to no effect on the activity of proteins or polypeptides.

As used herein, the term "modified protein" or "variant" refers to a polypeptide in which one or more amino acids are conservatively substituted and/or modified so as to be different from the amino acid sequence of the variant before modification, while retaining functions or properties. Such a variant may generally be identified by modifying one or more amino acids in the amino acid sequence of the polypeptide and evaluating the properties of the modified polypeptide. In other words, the ability of the variant may be increased, unchanged, or decreased compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "protein variant" may also be used interchangeably with modification, modified polypeptide, modified protein, mutant, mutein, divergent, etc., and any term is not limited, as long as it is used in a sense of being mutated. With respect to the objects of the present disclosure, the variant may be a polypeptide in which the amino acid corresponding to position 123 in the amino acid sequence of SEQ ID NO: 1 is substituted with an amino acid selected from the group consisting of alanine, cysteine, aspartic acid, glutamic acid, phenylalanine, glycine, histidine, isoleucine, lysine, leucine, methionine, asparagine, proline, glutamine, arginine, serine, threonine, tryptophan, and tyrosine, specifically, a polypeptide in which the amino acid is substituted with an amino acid selected from the group consisting of alanine, cysteine, aspartic acid, phenylalanine, glycine, histidine, isoleucine, leucine, methionine, asparagine, glutamine, serine, threonine, tryptophan and tyrosine. For example, the variant may have or comprise an amino acid sequence having 60% or more, 65% or more, 70% or more, 75% or more, 80% or more, 85% or more, 90% or more, 95% or more, 96% or more, 97% or more, 98% or more or 99% or more homology or identity to SEQ ID NO: 1, or may comprise or consist of an amino acid sequence having 70% or more, 75% or more, 80% or more, 85% or more, 90% or more, 95% or more, 96% or more, 97% or more, 98% or more or 99% or more, and less than 100% homology or identity to the sequence, or may essentially consist of the amino acid sequence. For a more specific example, the variant may be a polypeptide comprising an amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25 or SEQ ID NO: 27 or SEQ ID NO: 29 or SEQ ID NO: 31 or SEQ ID NO: 33 or SEQ ID NO: 35 or SEQ ID NO: 37 or SEQ ID NO: 39, but is not limited thereto.

In the threonine/homoserine export protein variant of the present disclosure, the amino acid corresponding to position 193 of SEQ ID NO: 1 may be isoleucine, alanine, cysteine, glycine, histidine, lysine, leucine, threonine, valine, or tryptophan. For example, in the threonine/homoserine export protein variant of the present disclosure, the amino acid corresponding to position 193 of SEQ ID NO: 1 may be isoleucine or valine, but is not limited thereto.

The variant may also comprise deletion or addition of amino acids that have minimal influence on the properties and secondary structure of the polypeptide. For example, a signal (or leader) sequence which co-translationally or post-translationally directs translocation of the protein may be conjugated to the N-terminus of the variant. The variant may also be conjugated to another sequence or a linker for identification, purification, or synthesis.

Further, the polynucleotide encoding the threonine/homoserine export protein variant of the present disclosure may comprise a nucleotide sequence encoding the threonine/homoserine export protein variant, in which the amino acid corresponding to position 123 in the amino acid sequence of SEQ ID NO: 1 is substituted with a different amino acid. For example, the polynucleotide of the present disclosure may comprise a nucleotide sequence encoding an amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25 or SEQ ID NO: 27 or SEQ ID NO: 29 or SEQ ID NO: 31 or SEQ ID NO: 33 or SEQ ID NO: 35 or SEQ ID NO: 37 or SEQ ID NO: 39. For example, the polynucleotide may have or comprise a nucleotide sequence having 60% or more, 65% or more, 70% or more, 75% or more, 80% or more, 85% or more, 90% or more, 95% or more, 96% or more, 97% or more, 98% or more or 99% or more homology or identity to SEQ ID NO: 2, or may comprise or consist of a nucleotide sequence having 70% or more, 75% or more, 80% or more, 85% or more, 90% or more, 95% or more, 96% or more, 97% or more, 98% or more or 99% or more, and less than 100% homology or identity to the sequence, or may essentially consist of the nucleotide sequence. For a more specific example of the present disclosure, the polynucleotide encoding the threonine/homoserine export protein variant of the present disclosure may have or comprise a sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26 or SEQ ID NO: 28 or SEQ ID NO: 30 or SEQ ID NO: 32 or SEQ ID NO: 34 or SEQ ID NO: 36 or SEQ ID NO: 38 or SEQ ID NO: 40. Further, the polynucleotide encoding the threonine/homoserine export protein variant of the present disclosure may consist of or essentially consist of a sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26 or SEQ ID NO: 28 or SEQ ID NO: 30 or SEQ ID NO: 32 or SEQ ID NO: 34 or SEQ ID NO: 36 or SEQ ID NO: 38 or SEQ ID NO: 40.

The polynucleotide of the present disclosure may undergo various modifications in the coding region without changing the amino acid sequence of the threonine/homoserine export protein variant of the present disclosure in consideration of codon degeneracy or the codons preferred in an organism that is intended to express the threonine/homoserine export protein variant of the present disclosure. Specifically, the polynucleotide encoding the threonine/homoserine export protein variant of the present disclosure may have or comprise a nucleotide sequence having 60%, 65%, 70% or more, 75% or more, 80% or more, 85% or more, 90% or more, 95% or more, 96% or more, 97% or more, 98% or more or 99% or more homology or identity to a sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26 or SEQ ID NO: 28 or SEQ ID NO: 30 or SEQ ID NO: 32 or SEQ ID NO: 34 or SEQ ID NO: 36 or SEQ ID NO: 38 or SEQ ID NO: 40, or may consist of or essentially consist of a nucleotide sequence having 70% or more, 75% or more, 80% or more, 85% or more, 90% or more, 95% or more, 96% or more, 97% or more, 98% or more or 99% or more homology or identity to the sequence, but is not limited thereto. In this regard, in the sequence having the homology or identity, the codon encoding the amino acid corresponding to position 123 of SEQ ID NO: 1 may be one of codons encoding an amino acid other than proline, for example, an amino acid selected from the group consisting of alanine, cysteine, aspartic acid, glutamic acid, phenylalanine, glycine, histidine, isoleucine, lysine, leucine, methionine, asparagine, glutamine, arginine, serine, threonine, tryptophan, and tyrosine, specifically, one of codons encoding an amino acid selected from the group consisting of alanine, cysteine, aspartic acid, phenylalanine, glycine, histidine, isoleucine, leucine, methionine, asparagine, glutamine, serine, threonine, tryptophan, and tyrosine.

As used herein, the term "corresponding to" refers to an amino acid residue at a position listed in a polypeptide, or an amino acid residue that is similar, identical or homologous to a residue listed in a polypeptide. Identifying the amino acid at the corresponding position may be determining a specific amino acid in a sequence that refers to a specific sequence. "Corresponding region" used herein generally refers to a similar or corresponding position in a related protein or reference protein.

For example, any amino acid sequence is aligned with SEQ ID NO: 1, and based on this, each amino acid residue of the amino acid sequence may be numbered by reference to the numerical position of the amino acid residue corresponding to the amino acid residue of SEQ ID NO: 1. For example, a sequence alignment algorithm such as that described herein may confirm the position of an amino acid, or a position where a modification such as substitution, insertion or deletion occurs, compared to a query sequence (also referred to as a "reference sequence").

For such alignment, for example, the Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277), and the like may be used without being limited thereto, and sequence alignment programs, pairwise sequence comparison algorithms, and the like known in the art may be appropriately used.

As used herein, the term "threonine/homoserine export protein (threonine/homoserine exporter, RhtA)" refers to a protein with threonine and homoserine export activity. Specifically, the "threonine/homoserine export protein" of the present disclosure may be used interchangeably with "RhtA". The threonine/homoserine export protein is known in the art, and specifically, may be one of NCBI Accession Nos. WP_264865234.1, WP_161647838.1, and WP_179150183.1, but is not limited thereto. The amino acid and polynucleotide sequences of the threonine/homoserine export protein may be obtained from known databases, for example, NCBI GenBank, etc., but are not limited thereto.

For example, the threonine/homoserine export protein may comprise the amino acid sequence of SEQ ID NO: 1 or an amino acid sequence having 60% or more homology or identity thereto, but is not limited thereto, as long as it has the threonine/homoserine export protein activity. Specifically, the polypeptide having the threonine/homoserine export protein activity may have or comprise SEQ ID NO: 1 or an amino acid sequence having at least 60%, 70%, 80%, 85%, 90%, 95%, 96%, 97%, 98%, or 99% or more homology or identity to SEQ ID NO: 1, or may consist of the amino acid sequence, or may essentially consist of the amino acid sequence. For example, the threonine/homoserine export protein may refer to a protein endogenously present in a microorganism of the genus *Corynebacterium* or *Corynebacterium glutamicum,* but is not limited thereto. Specifically, the threonine/homoserine export protein may be a threonine/homoserine export protein consisting of the amino acid sequence of SEQ ID NO: 1, which is endogenously present in a microorganism of the genus *Corynebacterium* or *Corynebacterium glutamicum,* but is not limited thereto.

Further, the threonine/homoserine export protein having the amino acid sequence of SEQ ID NO: 1 may be encoded by a polynucleotide having or comprising a sequence of SEQ ID NO: 2 or a nucleotide sequence having 60% or more, 70% or more, 75% or more, 80% or more, 85% or more, 90% or more, 95% or more, 96% or more, 97% or more, 98% or more, or 99% or more homology or identity to the sequence of SEQ ID NO: 2, or consisting of the nucleotide sequence, or essentially consisting of the nucleotide sequence, but is not limited thereto. Further, the nucleotide sequence of SEQ ID NO: 2 may be obtained from known databases, for example, NCBI GenBank, etc., but is not limited thereto.

In the present disclosure, the gene comprising the nucleotide sequence of SEQ ID NO: 2 may be used interchangeably with a polynucleotide comprising the nucleotide sequence of SEQ ID NO: 2, and a gene or polynucleotide having the nucleotide sequence of SEQ ID NO: 2.

The polynucleotide of the present disclosure may undergo various modifications in the coding region without changing the amino acid sequence of the threonine/homoserine export protein of the present disclosure due to codon degeneracy or in consideration of the codons preferred in an organism that is intended to express the threonine/homoserine export protein of the present disclosure. Therefore, it is apparent that a polynucleotide which may be translated to the polypeptide consisting of the amino acid sequence of the threonine/homoserine export protein of the present disclosure or a polypeptide having homology or identity thereto due to codon degeneracy may also be comprised. For example, the polynucleotide encoding the threonine/homoserine export protein of the present disclosure may be SEQ ID NO: 2 or a degenerated sequence thereof.

For another example, the polynucleotide encoding the threonine/homoserine export protein of the present disclosure may have or comprise a nucleotide sequence having 60% or more, 70% or more, 75% or more, 80% or more, 85% or more, 90% or more, 95% or more, 96% or more, 97% or more, 98% or more, or 99% or more homology or identity to SEQ ID NO: 2, or may consist of or essentially consist of a nucleotide sequence having 60% or more, 70% or more, 75% or more, 80% or more, 85% or more, 90% or more, 95% or more, 96% or more, 97% or more, 98% or more, or 99% or more homology or identity to SEQ ID NO: 2, but is not limited thereto.

Further, the polynucleotide of the present disclosure may comprise a probe which may be prepared from a known nucleotide sequence, for example, any sequence without limitation as long as it hybridizes with a sequence complementary to the entirety or a part of the polynucleotide sequence of the present disclosure under stringent conditions to encode the threonine/homoserine export protein of the present disclosure.

Still another aspect of the present disclosure provides a vector comprising the polynucleotide of the present disclosure.

The vector may be an expression vector for expressing the polynucleotide in a host cell, but is not limited thereto.

The vector is as described above.

Still another aspect of the present disclosure provides a microorganism comprising any one or more selected from the threonine/homoserine export protein variant of the present disclosure; the polynucleotide encoding the same; or a vector comprising the polynucleotide.

The microorganism of the present disclosure may have an L-amino acid-producing ability.

In the present disclosure, the "L-amino acid" comprises all of L-homoserine, L-homoserine derivatives, and L-amino acids biosynthesized using L-homoserine as a precursor.

In the present disclosure, the "L-homoserine derivatives" may be, for example, O-acetyl-L-homoserine, O-succinyl-L-homoserine, etc., but are not necessarily limited thereto, and any derivative which is obtained through a fermentation process and has a substituent linked to the terminal oxygen of L-homoserine in the art may fall within the scope of the present disclosure.

In the present disclosure, "L-amino acids biosynthesized using L-homoserine as a precursor" may comprise, for example, methionine, threonine, isoleucine, etc., in which L-homoserine is employed as a precursor, but is not necessarily limited thereto, and any L-amino acid biosynthesized using L-homoserine as a precursor may fall within the scope of the present disclosure.

For example, the "L-amino acid" may be any one or more selected from the group consisting of L-homoserine, O-acetyl-L-homoserine, O-succinyl-L-homoserine, L-methionine, and L-threonine.

With respect to the objects of the present disclosure, the microorganism of the present disclosure may be a microorganism comprising the threonine/homoserine export protein variant or the polynucleotide encoding the same; or a microorganism (e.g., recombinant strain) genetically modified to comprise the threonine/homoserine export protein variant or the polynucleotide encoding the same.

The strain of the present disclosure may be a strain naturally having the L-amino acid-producing ability or a microorganism prepared by providing the L-amino acid-producing ability for a strain without the L-amino acid-producing ability. For example, the strain may be a microorganism in which the L-amino acid-producing ability is increased by introducing the threonine/homoserine export protein variant of the present disclosure or the polynucleotide encoding the same, but is not limited thereto.

The strain of the present disclosure may be a microorganism having increased L-amino acid-producing ability compared to a parent strain without the variant of the present disclosure or a wild-type strain of the genus *Mycobacterium.* The microorganism may have the improved L-amino acid-producing ability by introducing the threonine/homoserine export protein variant of the present disclosure having increased threonine/homoserine export activity compared to the wild-type threonine/homoserine export protein, or a polynucleotide encoding the same. Specifically, the strain of the present disclosure may have increased L-amino acid-producing ability compared to a microorganism of the genus *Corynebacterium* comprising the wild-type threonine/homoserine export protein having the amino acid sequence of SEQ ID NO: 1 or the polynucleotide encoding the same.

For example, the microorganism having the L-amino acid-producing ability refers to a prokaryotic or eukaryotic microbial strain capable of producing L-amino acids within a living organism, and may comprise both a microorganism inherently having the L-amino acid-producing ability or a microorganism prepared by providing the L-amino acid-producing ability for a parent strain without the L-amino acid-producing ability due to the increased threonine/homoserine export protein activity, in which the parent strain is genetically modified to comprise the threonine/homoserine export protein variant of the present disclosure or the polynucleotide encoding the same. The L-amino acid-producing ability may be conferred or enhanced by species improvement.

For example, the recombinant microorganism having the L-amino acid-producing ability of the present disclosure may comprise any microorganism capable of producing L-amino acids, which is transformed with a vector to express the threonine/homoserine export protein variant of the present disclosure having increased threonine/homoserine export protein activity.

For example, the microorganism producing L-amino acids may be a microorganism inherently comprising the protein consisting of the amino acid sequence of SEQ ID NO: 1, or a protein consisting of an amino acid sequence having at least 60%, 70%, 75%, 80%, 85%, 90%, 95%, 96%, 97%, 98%, 99%, 99.5%, 99.7% or 99.9% or more homology or identity to SEQ ID NO: 1.

For example, the microorganism producing L-amino acids may be a microorganism inherently comprising a polynucleotide sequence capable of encoding a protein comprising an amino acid sequence having at least 80% homology to SEQ ID NO: 1, the nucleotide sequence of SEQ ID NO: 2, or a nucleotide sequence having 60% or more, 70% or more, 75% or more, 80% or more, 85% or more, 90% or more, 95% or more, 96% or more, 97% or more, 98% or more or 99% or more homology or identity to the nucleotide sequence of SEQ ID NO: 2.

The microorganism of the present disclosure may comprise any microorganism with increased threonine/homoserine export protein activity compared to its endogenous activity, by expressing the threonine/homoserine export protein variant of the present disclosure having increased threonine/homoserine export protein activity by various known methods.

In one specific embodiment, the microorganism with increased threonine/homoserine export protein activity of the present disclosure compared to its endogenous activity, may be a microorganism in which the amino acid sequence of the threonine/homoserine export protein or the polynucleotide sequence encoding the same is modified, but is not limited thereto. The modification of the amino acid sequence or polynucleotide sequence may comprise all of deletion, substitution, insertion, etc. The modification on the sequence is as described above. In another specific embodiment, the microorganism with increased threonine/homoserine export protein activity of the present disclosure compared to its endogenous activity, may be a microorganism in which the gene expression regulatory region (or expression regulatory sequence) on the chromosome encoding the threonine/homoserine export protein is replaced with a sequence with strong activity, but is not limited thereto. The expression regulatory region may be, for example, a promoter. The replacement with a sequence with strong activity is as described above. In still another specific embodiment, in the microorganism with increased threonine/homoserine export protein activity of the present disclosure compared to its endogenous activity, the increase of the threonine/homoserine export protein activity may be achieved by a combination of the above specific embodiments.

In one embodiment of the present disclosure, the microorganism of the present disclosure may have the L-amino acid-producing ability.

The increased threonine/homoserine export protein activity may be defined as, but is not limited to, increased L-amino acid-producing ability of the microorganism of the present disclosure compared to the production ability of a native wild-type microorganism or an unmodified microorganism (e.g., a microorganism expressing the polypeptide having the wild-type threonine/homoserine export protein activity (e.g., the polypeptide of SEQ ID NO: 1) or a strain in which the threonine/homoserine export protein activity of the present disclosure is not increased or has not yet been increased, compared to the endogenous activity).

For example, the threonine/homoserine export protein activity may be determined by measuring the L-amino acid-producing ability or yield, but is not limited thereto. For example, the threonine/homoserine export protein activity may be determined according to the L-amino acid-producing ability described in Example 2-4, but is not limited thereto.

For example, the microorganism with increased L-amino acid-producing ability of the present disclosure may be a microorganism with increased L-amino acid-producing ability compared to an unmodified microorganism, but is not limited thereto. For example, the unmodified microorganism which is a subject strain for comparing whether or not the L-amino acid-producing ability is increased may be a strain of *Corynebacterium glutamicum* (Cgl-HS-2) having hom mutations (G378E, R398Q) and a lysC mutation (L377K) and a strain of *Corynebacterium glutamicum* (Cgl-HS-3) in which a promoter of the threonine/homoserine export protein is replaced with a gapA promoter, but is not limited thereto.

For example, the microorganism with increased L-amino acid-producing ability may have an increased L-amino acid-producing ability of about 1% or more, specifically, about 1% or more, about 10% or more, about 11.1% or more, about 20% or more, about 22.2% or more, about 30% or more, about 33.3% or more, about 40% or more, about 44.4% or more, about 50% or more, about 55.6% or more, about 60% or more, about 70% or more, about 80% or more, about 90% or more, about 100% or more, about 110% or more, about 120% or more, about 130% or more, about 140% or more, about 150% or more, about 160% or more, about 170% or more, about 177.8% or more, about 180% or more, about 190% or more, about 200% or more, about 210% or more, about 220% or more, about 222.2% or more, about 230% or more, about 233.3% or more, about 240% or more, about 250% or more or about 255.6% or more (the upper limit is not particularly limited, but may be, for example, about 300% or less, about 200% or less, about 150% or less, about 100% or less, about 50% or less, about 45% or less, about 40% or less, about 35% or less, about 30% or less, about 25% or less, about 20% or less or about 15% or less), as compared to the L-amino acid-producing ability of the parent strain before modification or the unmodified microorganism, but the increased amount is not limited thereto as long as the production ability has an increased amount of a + value, as compared to the production ability of the parent strain before modification or the unmodified microorganism. In another example, the recombinant strain with increased L-amino acid-producing ability may have an increased L-amino acid-producing ability of about 1.01 times or more, about 1.1 times or more, about 1.111 times or more, about 1.2 times or more, about 1.222 times or more, about 1.3 times or more, about 1.333 times or more, about 1.4 times or more, about 1.444 times or more, about 1.5 times or more, about 1.556 times or more, about 1.6 times or more, about 1.7 times or more, about 1.8 times or more, about 1.9 times or more, about 2 times or more, about 2.1 times or more, about 2.2 times or more, about 2.3 times or more, about 2.4 times or more, about 2.5 times or more, about 2.6 times or more, about 2.7 times or more, about 2.778 times or more, about 2.8 times or more, about 2.9 times or more, about 3 times or more, about 3.1 times or more, about 3.2 times or more, about 3.222 times or more, about 3.3 times or more, about 3.333 times or more, about 3.4 times or more, about 3.5 times or more or about 3.556 times or more (the upper limit is not particularly limited, but may be, for example, about 10 times or less, about 5 times or less, about 3 times or less, about 2 times or less, about 1.5 times or less or about 1.2 times or less), as compared to the parent strain before modification or the unmodified microorganism, but is not limited thereto.

For still another example of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium glutamicum,* but is not limited thereto.

Meanwhile, it was already known that microorganisms of the genus *Corynebacterium* are able to produce, as L-amino acids, L-homoserine, L-homoserine derivatives, or L-amino acids biosynthesized using L-homoserine as a precursor, but their productivity is significantly low, and the genes and mechanisms involved in the production mechanism have not been fully elucidated. Therefore, the microorganism of the genus *Corynebacterium* of the present disclosure having the ability to produce L-homoserine, L-homoserine derivatives, or L-amino acids biosynthesized using L-homoserine as a precursor may comprise all of a natural wild-type microorganism itself, a microorganism of the genus *Corynebacterium* that has the enhanced ability to produce L-homoserine, L-homoserine derivatives, or L-amino acids biosynthesized using L-homoserine as a precursor by increasing or decreasing the activities of genes related to the production mechanism of L-homoserine, L-homoserine derivatives, or L-amino acids biosynthesized using L-homoserine as a precursor, or a microorganism of the genus *Corynebacterium* that has the enhanced ability to produce L-homoserine, L-homoserine derivatives, or L-amino acids biosynthesized using L-homoserine as a precursor by introducing or increasing the activity of a foreign gene.

The microorganism having the L-amino acid-producing ability of the present disclosure may be a microorganism in which homoserine dehydrogenase or lysine-sensitive aspartokinase 3 activity in the biosynthetic pathway of L-homoserine, L-homoserine derivatives, or L-amino acids biosynthesized using L-homoserine as a precursor is further increased or decreased, or a microorganism with the enhanced ability to produce productivity of L-homoserine, L-homoserine derivatives, or L-amino acids biosynthesized using L-homoserine as a precursor, but is not limited thereto.

Examples of the mutation of the homoserine dehydrogenase or lysine-sensitive aspartokinase 3 which may be applied to the present disclosure are disclosed in Korean Patent No. 10-2183209 or Korean Patent Publication No. 10-2019-0003019, respectively, the disclosure of which is hereby incorporated by reference herein in its entirety.

Still another aspect of the present disclosure provides a method of producing an L-amino acid, the method comprising the step of culturing, in a medium, the microorganism comprising any one or more selected from the threonine/homoserine export protein variant of the present disclosure; the polynucleotide encoding the same; or a vector comprising the polynucleotide.

With regard to the method of the present disclosure, the culturing of the microorganism may be performed using any culturing conditions and culturing method known in the art. Such culturing procedure may be easily adjusted for use by a person skilled in the art according to the selected strain.

The L-amino acid produced by culturing of the present disclosure may be released into the medium or may remain in cells.

The L-amino acid may be any one or more selected from the group consisting of L-homoserine, O-acetyl-L-homoserine, O-succinyl-L-homoserine, L-methionine, and L-threonine, which are as described above.

In one specific embodiment, the method of producing an L-amino acid of the present disclosure may further comprise the steps of preparing the microorganism of the present disclosure, preparing a medium for culturing the strain, or a combination of these steps (regardless of the order, in any order), for example, before the culturing step.

The method of producing an L-amino acid of the present disclosure may further comprise the step of recovering a desired substance, specifically, the L-amino acid from the cultured microorganism, a culture of the microorganism, a fermentation product of the microorganism, or the culture medium. The recovering step may be further comprised after the culturing step.

The recovering may be collecting a desired L-amino acid by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, affinity chromatography, HPLC, and a combination of these methods may be used, and a desired substance, specifically, an L-amino acid may be recovered from the medium or microorganism by using an appropriate method known in the art.

In addition, the method of producing an L-amino acid of the present disclosure may further comprise a purification step. The purification may be performed by using an appropriate method known in the art. In an exemplary embodiment, when the method of producing an L-amino acid of the present disclosure comprises both the recovering step and the purification step, the recovering step and the purification step may be performed continuously or discontinuously regardless of the order, or may be performed simultaneously or integrated into one step, but is not limited thereto.

With regard to the method of the present disclosure, the threonine/homoserine export protein variant and L-amino acid, etc. are as described in other aspects.

Still another aspect of the present disclosure provides a composition for producing an L-amino acid, the composition comprising the microorganism comprising any one or more selected from the threonine/homoserine export protein variant of the present disclosure; the polynucleotide encoding the same; or a vector comprising the polynucleotide, a culture of the microorganism, a fermentation product of the microorganism, or a combination of two or more thereof.

The composition of the present disclosure may further comprise any appropriate excipient that is usually used in the composition for producing L-amino acids, and examples of the excipient may comprise a preserving agent, a wetting agent, a dispersing agent, a suspending agent, a buffer, a stabilizing agent, an isotonic agent, etc., but are not limited thereto.

In a specific embodiment, each component present in the composition of the present disclosure may be comprised in a microbially effective amount, or in an amount that may be appropriately present in the composition for production.

With regard to the composition of the present disclosure, the threonine/homoserine export protein variant and L-amino acid, etc. are as described in other aspects.

Still another aspect of the present disclosure provides use of the threonine/homoserine export protein variant of the present disclosure in producing an L-amino acid.

Still another aspect of the present disclosure provides use of the microorganism comprising any one or more selected from the threonine/homoserine export protein variant of the present disclosure; the polynucleotide encoding the same; or the vector comprising the polynucleotide in producing an L-amino acid.

With regard to the use of the present disclosure, the threonine/homoserine export protein variant and L-amino acid, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Screening for threonine/homoserine export protein (RhtA) mutant library

### Example 1-1. Preparation of RhtA mutant library and Preparation of strain for screening

To prepare a template to be used in error-prone PCR, a wild-type rhtA gene fragment encoding RhtA was obtained by performing PCR using the genomic DNA of *Escherichia coli* W3110 strain as a template and a pair of primers of SEQ ID NO: 41 and SEQ ID NO: 42. In addition, a promoter fragment of the rhtB gene was obtained by performing PCR using the genomic DNA of *E*. *coli* W3110 strain as a template and a pair of primers of SEQ ID NO: 43 and SEQ ID NO: 44.

PCR reaction was performed using Solg^{™} Pfu-X DNA polymerase under conditions of denaturation at 95°C for 5 minutes, followed by 30 cycles of denaturation at 95°C for 30 seconds, annealing at 60°C for 30 seconds, polymerization at 72°C for 60 seconds, and then polymerization at 72°C for 5 minutes.

The primer sequences used here are as shown in Table 1 below.

**[Table 1]**

| SEQ ID NO. | Sequence name | Sequence (5'→3') |
|---|---|---|
| 41 | rhtA F | AGACACACCGGGAGTTCATCATGCCTGG TTCATTACGT |
| 42 | rhtA R | AATTCGAGCTCGGTACCCTTAATTAATGT CTAATTCT |
| 43 | PrhtB F | GTCGACTCTAGAGGATCCCCCGGGTAAT CTGCCTCTTA |
| 44 | PrhtB R | ACGTAATGAACCAGGCATGATGAACTCCC GGTGTGTCT |

The gene fragments obtained above were cloned into a pCL1920 vector (Nucleic Acids Rersearch, 18, (1990) 4631) digested with Smal restriction enzyme using the Gibson assembly method (DG Gibson et al., NATURE MEHSODS, VOL.6 NO.5, MAY 2009, NEBuilder HiFi DNAAssembly Master Mix) to obtain a recombinant plasmid pCL1920-PrhtB_rhtA(wt). Cloning was performed by mixing the Gibson assembly reagent and each gene fragment in the calculated numbers of moles and then storing the mixture at 50°C for 1 hour.

Error-prone PCR was performed to induce random mutagenesis in the wild-type rhtA gene encoding the threonine/homoserine export protein, and a diversify PCR random mutagenesis kit (Takara) was used in performing the error-prone PCR. To select the mutation rate conditions, error-prone PCR was performed under two conditions according to the addition amount of MnSO₄, as shown below. PCR was performed using pCL1920-PrhtB_rhtA (wt) as a DNA template into which mutations were intended to be introduced, and a pair of primers of SEQ ID NO: 41 and SEQ ID NO: 42. PCR was performed under conditions of denaturation at 95°C for 30 seconds, followed by 25 cycles of denaturation at 95°C for 30 seconds, annealing at 55°C for 30 seconds, polymerization at 68°C for 30 seconds, and then polymerization at 68°C for 60 seconds.

The compositions for performing the error-prone PCR were as shown in Table 2 below.

**[Table 2]**

| Case # | Case #1(MnSO₄ 1 µl) | Case #2(MnSO₄ 2 µl) |
|---|---|---|
| 10X Titanium taq Buffer | 5 | 5 |
| MnSO₄(8mM) | 1 | 2 |
| dGTP (2mM) | 1 | 1 |
| 50 X dNTP Mix | 1 | 1 |
| Titanium Taq Polymerase | 1 | 1 |
| Forward primer(5pmol) | 2 | 2 |
| Reverse primer(5pmol) | 2 | 2 |
| Template DNA | 1 | 1 |
| dH₂O | 36 | 35 |
| Total | 50 | 50 |

The error-prone PCR product thus obtained was treated with Dpnl to remove the template plasmid, and the resulting DNA was cloned into a pCL1920 vector digested with Smal restriction enzyme using the Gibson assembly method to obtain a recombinant mutant plasmid library pCL1920-PrhtB_rhtA(mt).

### Example 1-2. Preparation of strain for productivity screening of RhtA mutant library

To prepare a W3110(△rhtABC) strain for library screening, PCR was first performed for the deletion of the rhtBC gene using genomic DNA of E. *coli* as a template and primer pairs of SEQ ID NO: 45 and SEQ ID NO: 46 and SEQ ID NO: 47 and SEQ ID NO: 48, thereby amplifying the rhtBC upstream and downstream homologous region fragments, respectively.

The rhtBC upstream and downstream homologous region fragments obtained through the above process were cloned into a pSKH130 vector digested with Smal restriction enzyme using the Gibson assembly method to obtain a recombinant plasmid pSHK130△rhtBC. Cloning was performed by mixing the Gibson assembly reagent and each gene fragment in the calculated numbers of moles and then storing the mixture at 50°C for 1 hour.

Next, PCR was performed for the deletion of the rhtA gene using genomic DNA of E. *coli* W3110 strain as a template and primer pairs of SEQ ID NO: 49 and SEQ ID NO: 50 and SEQ ID NO: 51 and SEQ ID NO: 52, thereby amplifying the rhtA upstream and downstream homologous region fragments, respectively.

The rhtA upstream and downstream homologous region fragments obtained through the above process were cloned into a pSKH130 vector digested with Smal restriction enzyme using the Gibson assembly method to obtain a recombinant plasmid pSHK130△rhtA.

PCR reaction was performed using Solg^{™} Pfu-X DNA polymerase under conditions of denaturation at 95°C for 5 minutes, followed by 30 cycles of denaturation at 95°C for 30 seconds, annealing at 58°C for 30 seconds, polymerization at 72°C for 60 seconds, and polymerization at 72°C for 5 minutes.

The above-prepared pSHK130△rhtA and pSHK130△rhtBC vectors were transformed into E. *coli* W3110 strain by electroporation (Appl. Microbiol. Biotechnol. (1999) 52:541-545), and through a secondary crossover process, a W3110(△rhtABC) strain with the deletion of the rhtABC gene on the chromosome was obtained.

The corresponding genetic manipulation was confirmed by performing PCR and genome sequencing using a pair of primers of SEQ ID NO: 53 and SEQ ID NO: 54, which are able to amplify the external regions of the homologous recombination upstream and downstream regions where the corresponding gene was inserted, respectively.

The primer sequences used here are as shown in Table 3 below.

**[Table 3]**

| SEQ ID NO. | Sequence name | Sequence (5'→3') |
|---|---|---|
| 45 | rhtBC up F | GCTGCAGGAATTCgatCCGCCAGATCATCA ACATA |
| 46 | rhtBC up R | TTTTTTGTCTCTCGATATCCCCAGCCAAAT CAAGTA |
| 47 | rhtBC down F | ACTTGATTTGGCTGGGGATATCGAGAGAC AAAAAAG |
| 48 | rhtBC down R | AGGTCGACTAGCGTGATAGGAGCCGTGCG GGAAC |
| 49 | rhtA up F | TGCAGGAATTCGATAAAGAACCGATCACAC CCAGCGG |
| 50 | rhtA up R | AGAGCGGTCGAGAGAATAGATATCTCCTTT CTCCCA |
| 51 | rhtA down F | TGGGAGAAAGGAGATATCTATTCTCTCGAC CGCTCT |
| 52 | rhtA down R | AGCTAGGTCGACTAGCGTGATGGCTATGT GTTCCG |
| 53 | rhtABC up F | GATTTAACTAATTTAGCGG |
| 54 | rhtABC up R | CCGCTTCTAATCACTTTGG |
| 55 | rhtABC down F | GGCTGAACCTCCTGCTGCC |
| 56 | rhtABC down R | CAGCAATACCAGACGACGG |

### Example 1-3. Productivity screening of RhtA mutant library

The mutant library obtained in Example 1-1, pCL1920-PrhtB_rhtA(mt), and pCL1920-PrhtB_rhtA(wt) were each transformed into the W3110(△rhtABC) strain and cultured on an LB plate medium comprising 50 µg/L spectinomycin. Fifty colonies were selected from the W3110(△rhtABC) strain transformed with the mutant library, and subjected to sequencing to determine the mutation rate and the presence or absence of mutations at various sites.

The sequencing results showed that the mutation rate under case #1 condition of Example 1-1 was 1.2 kb⁻¹ and the mutation rate under case #2 condition was 2.0 kb⁻¹. Accordingly, it was determined that both cases #1 and #2 satisfied the mutation rates suitable for securing a mutant library, and a procedure of screening for valid mutations was performed using the libraries produced under the above conditions.

The W3110(△rhtABC) strains, each transformed with pCL 1920-PrhtB_rhtA(mt) or pCL1920-PrhtB_rhtA(wt), were inoculated into a 96-Deep Well Plate-Dome (Bioneer) comprising 400 µL of a seed medium, respectively, and cultured in a plate shaking incubator (TAITEC) at 32°C and 12,000 rpm for about 12 hours.

### <Seed medium (pH 7.2)>

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium-pantothenic acid 2000 µg, Nicotinamide 2000 µg

For a primary screening of about 1,000 cultured colonies, each colony was serially diluted in the seed medium comprising 200 g/L of L-homoserine and tested for minimum inhibitory concentration (MIC). 29 types of colonies were obtained, which showed a significantly high MIC value, relative to the comparative strain, W3110 (△rhtABC) transformed with pCL 1920-PrhtB_rhtA(wt).

Each of the colonies obtained was inoculated into a 96-Deep Well Plate-Dome comprising 400 µL of the seed medium, and cultured for about 12 hours in a plate shaking incubator at 32°C and 12,000 rpm.

For a secondary screening of the 29 types of cultured colonies, the initial optical density (OD) values of the final cultured colonies were equalized, and serially diluted in the seed medium comprising 200 g/L of L-homoserine, and tested for minimum inhibitory concentration (MIC). 7 types of colonies were selected, which showing a significantly high MIC value, relative to the comparative strain W3110(△rhtABC) (K12/pCL1920-Pn_rhtA) transformed with pCL1920-PrhtB_rhtA(wt) (Table 4).

**[Table 4]**

| Name of strain | OD |
|---|---|
| K12/pCL1920-Pn_rhtA (pCL1920-PrhtB_rhtA(wt)) | 0.45 |
| K12/pCL1920-Pn_rhtA(m1) (pCL1920-PrhtB_rhtA(m1)) | 0.75 |
| K12/pCL1920-Pn_rhtA(m2) (pCL1920-PrhtB_rhtA(m2)) | 0.78 |
| K12/pCL1920-Pn_rhtA(m3) (pCL1920-PrhtB_rhtA(m3)) | 0.65 |
| K12/pCL1920-Pn_rhtA(m4) (pCL1920-PrhtB_rhtA(m4)) | 0.74 |
| K12/pCL1920-Pn_rhtA(m5) (pCL1920-PrhtB_rhtA(m5)) | 0.65 |
| K12/pCL1920-Pn_rhtA(m6) (pCL1920-PrhtB_rhtA(m6)) | 0.63 |
| K12/pCL1920-Pn_rhtA(m7) (pCL1920-PrhtB_rhtA(m7)) | 0.80 |

The mutant plasmid pCL1 920-Pn_rhtA was extracted from 7 types of selected colonies, and then sequencing was performed to examine the mutation, and as a result, it was confirmed that a mutation occurred in the coding sequence (CDS).

The mutant plasmids derived from 7 types of selected colonies were named pCL1920-PrhtB_rhtA(m1), pCL1920-PrhtB_rhtA(m2), pCL1920-PrhtB_rhtA(m3), pCL1920-PrhtB_rhtA(m4), pCL1920-PrhtB_rhtA(m5), pCL1920-PrhtB_rhtA(m6), and pCL1920-PrhtB_rhtA(m7), respectively. Specifically, it was confirmed that pCL1920-PrhtB_rhtA(m1) comprised rhtA(T260A) mutation, pCL1920-PrhtB_rhtA(m2) comprised rhtA(S183A) mutation, pCL1920-PrhtB_rhtA(m3) comprised rhtA(V123A/I193V) mutation, pCL1920-PrhtB_rhtA(m4) comprised rhtA(F66Y) mutation, pCL1920-PrhtB_rhtA(m5) comprised rhtA(V15A/A229V) mutation, pCL1920-PrhtB_rhtA(m6) comprised rhtA(L210S) mutation, and pCL1920-PrhtB_rhtA(m7) comprised rhtA(P119T) mutation.

### Example 1-4. Construction of RhtA mutant plasmid for introduction into Corynebacterium strain

For comparative evaluation of the RhtA activity by introducing the RhtA mutations selected in Example 1-3 into a *Corynebacterium* strain, rhtA mutant plasmids were prepared by the following method.

First, for homologous recombination, PCR was performed using genomic DNA of the wild-type *Corynebacterium glutamicum* ATCC13032 strain as a template and a pair of primers of SEQ ID NO: 57 and SEQ ID NO: 58 and a pair of primers of SEQ ID NO: 63 and SEQ ID NO: 64 to amplify the upstream and downstream regions of Ncgl0998, respectively. To utilize the gapA promoter as a promoter of the mutant rhtA gene, PCR was performed using genomic DNA of *Corynebacterium glutamicum* ATCC13032 as a template and a pair of primers of SEQ ID NO: 59 and SEQ ID NO: 60 to amplify the gapA promoter. In addition, PCR was performed using the mutant plasmids pCL1920-PrhtB_rhtA(m1), pCL 1920-PrhtB_rhtA(m2), pCL1920-PrhtB_rhtA(m3), pCL1920-PrhtB_rhtA(m4), pCL1920-PrhtB_rhtA(m5), pCL1920-PrhtB_rhtA(m6), pCL1920-PrhtB_rhtA(m7) obtained in Example 1-3 as templates and a pair of primers of SEQ ID NO: 61 and SEQ ID NO: 62 to amplify fragments of the mutant rhtA from the mutant plasmids selected in Example 1-3, respectively, thereby obtaining rhtA, rhtA(m1), rhtA(m2), rhtA(m3), rhtA(m4), rhtA(m5), rhtA(m6), and rhtA(m7) fragments.

The PCR reaction was performed using Solg^{™} Pfu-X DNA polymerase under conditions of denaturation at 95°C for 5 minutes, followed by 30 cycles of denaturation at 95°C for 30 seconds, annealing at 55°C for 30 seconds, polymerization at 72°C for 60 seconds, and then polymerization at 72°C for 5 minutes.

The primer sequences used here are as shown in Table 5 below.

**[Table 5]**

| SEQ ID NO. | Sequence name | Sequence (5'→3') |
|---|---|---|
| 57 | Ncgl0998 up F | CGAGCTCGGTACCCTAATAATTCTGCTT CCCGACG |
| 58 | Ncgl0998 up R | CCACAAATTAAGGGACGGTCGATGTCGA GG |
| 59 | PgapA F | CTCGACATCGACCGTCCCTTAATTTGTG GATTCAC |
| 60 | PgapA R | CGTAATGAACCAGGCATGTTGTGTCTCC TCTAAA |
| 61 | rhtA_m F | TTAGAGGAGACACAACATGCCTGGTTCA TTACGTA |
| 62 | rhtA_m R | TCGGGCTCTTCCGGTTCCCTTAATTAATG TCTAAT |
| 63 | Ncgl0998 dn F | AGACATTAATTAAGGGAACCGGAAGAGC CCGATTG |
| 64 | Ncgl0998 dn R | TAGAGGATCCCCGAAAAGGAAGAAGTCT GAGAAGAAGG |

The gene fragments obtained through the above process were each cloned into a pDCM2 vector (Korean Patent Publication No. 10-2020-0136813) digested with Smal restriction enzyme using the Gibson assembly method to obtain 8 types of recombinant plasmids comprising the wild-type rhtA or the mutant rhtA. Cloning was performed by mixing the Gibson assembly reagent and each gene fragment in the calculated numbers of moles and then storing the mixture at 50°C for 1 hour. The 8 types of constructed recombinant plasmids were named pDCM2-PgapA_rhtA, pDCM2-PgapA_rhtA(m1), pDCM2-PgapA_rhtA(m2), pDCM2-PgapA_rhtA(m3), pDCM2-PgapA_rhtA(m4), pDCM2-PgapA_rhtA(m5), pDCM2-PgapA_rhtA(m6), and pDCM2-PgapA_rhtA(m7), respectively.

### Example 2. Preparation of L-homoserine-producing strain

### Example 2-1. Preparation of homoserine dehydrogenase (hom) mutant strain

L-homoserine production was intended to be increased by introducing, into a *Corynebacterium* strain, mutation (G378E, R398Q) traits (Korean Patent No. 10-2183209) for releasing the feedback inhibition of hom by L-homoserine. For this purpose, a hom mutation plasmid was constructed by the following method.

First, a fragment of the promoter upstream region of the hom gene was amplified by performing PCR using genomic DNA of the wild-type *Corynebacterium glutamicum* ATCC13032 strain as a template and a pair of primers of SEQ ID NO: 65 and SEQ ID NO: 66.

Further, in order to apply the hom mutations (G378E, R398Q), the upstream fragment of the gene encoding the CDS comprising amino acids at positions 1 to 378 from the N-terminus of an amino acid sequence of the hom protein, the hom G378E/R398Q gene fragment, and the downstream fragment of the hom R398Q gene where homologous recombination occurs on the chromosome were obtained. In detail, PCR was performed using genomic DNA of the wild-type *Corynebacterium glutamicum* ATCC13032 strain as a template and a pair of primers of SEQ ID NO: 67 and SEQ ID NO: 68, a pair of primers of SEQ ID NO: 69 and SEQ ID NO: 70, and a pair of primers of SEQ ID NO: 71 and SEQ ID NO: 72 to obtain the upstream fragment of the coding sequence comprising amino acids at positions 1 to 378 from the N-terminus of the amino acid sequence of the hom protein, the G378E/R398Q gene fragment, and the downstream fragment of the R398Q gene, respectively.

PCR reaction was performed using Solg^{™} Pfu-X DNA polymerase under conditions of denaturation at 95°C for 5 minutes, followed by 30 cycles of denaturation at 95°C for 30 seconds, annealing at 55°C for 30 seconds, polymerization at 72°C for 60 seconds, and then polymerization at 72°C for 5 minutes.

The gene fragments obtained through the above process were cloned into pDCM2 vector digested with Smal restriction enzyme using the Gibson assembly method to obtain a recombinant plasmid. Cloning was performed by mixing the Gibson assembly reagent and each gene fragment in the calculated numbers of moles and then storing the mixture at 50°C for 1 hour. The constructed recombinant plasmid was named pDCM2-hom(G378E, R398Q).

The pDCM2-hom(G378E, R398Q) vector constructed above was transformed into the wild-type *Corynebacterium glutamicum* ATCC13032 strain by electroporation, and through a secondary crossover process, a strain was obtained, in which the wild-type hom gene on the chromosome was replaced with the mutant hom (G378E, R398Q) gene.

The genetic manipulation was confirmed by performing PCR and genome sequencing using a pair of primers of SEQ ID NO: 73 and SEQ ID NO: 74, which are able to amplify the external regions of the homologous recombination upstream and downstream regions where the corresponding gene was inserted, respectively.

The primer sequences used here are as shown in Table 6 below.

**[Table 6]**

| SEQ ID NO. | Sequence name | Sequence (5'→3') |
|---|---|---|
| 65 | up F | TCGAGCTCGGTACCCTGTCTCCGTATGCA GTGAGC |
| 66 | up R | GGATGTTTCTTTGGAGCTTCGCTCAATCA T |
| 67 | hom F | TATAGGAGAACAATCATGACCTCAGCATC TGCCCC |
| 68 | G378E R | GCCAAAACCTCCACGCGATCTT |
| 69 | G378E F | AAGATCGCGTGGAGGTTTTGGC |
| 70 | R398Q R | GCGCTCTTCCTGTTGGATTGTACGC |
| 71 | R398Q F | GCGTACAATCCAACAGGAAGAGCGC |
| 72 | hom R | CTCTAGAGGATCCCCGACTGCGGAATGTT GTTGTG |
| 73 | hom conf F | TGGGTAGGTCGAGTTGTTAA |
| 74 | hom conf R | CAGCGCAGTCGCACGAATAT |

The transformed strain obtained above was named Cgl-HS-1.

### Example 2-2. Preparation of lysine-sensitive aspartokinase 3 (LysC) mutant strain

It was intended to introduce, into a *Corynebacterium* strain, a mutation (L377K) trait (Korean Patent Publication No. 10-2019-0003019) for enhancing lysC gene expression and releasing the feedback inhibition by L-lysine and L-homoserine.

First, the upstream region of the lysC gene promoter, where homologous recombination on the chromosome occurs, and the upstream and downstream regions of the lysC L377K gene were obtained. In detail, PCR was performed using genomic DNA of the wild-type *Corynebacterium glutamicum* ATCC13032 strain as a template, and a pair of primers of SEQ ID NO: 75 and SEQ ID NO: 76, a pair of primers of SEQ ID NO: 77 and SEQ ID NO: 78, and a pair of primers of SEQ ID NO: 79 and SEQ ID NO: 80 to obtain gene fragments of the upstream region of the lysC gene promoter, the downstream region of the lysC L377K gene, and the upstream region of the lysC L377K gene, respectively.

PCR reaction was performed using Solg^{™} Pfu-X DNA polymerase under conditions of denaturation at 95°C for 5 minutes, followed by 30 cycles of denaturation at 95°C for 30 seconds, annealing at 60°C for 30 seconds, polymerization at 72°C for 60 seconds, and then polymerization at 72°C for 5 minutes.

The gene fragments obtained through the above process were cloned into a pDCM2 vector digested with Smal restriction enzyme using the Gibson assembly method to obtain a recombinant plasmid. Cloning was performed by mixing the Gibson assembly reagent and each gene fragment in the calculated numbers of moles and then storing the mixture at 50°C for 1 hour. The constructed recombinant plasmid was named pDCM2-_IysC L377K.

The pDCM2-_IysC L377K vector constructed above was transformed into the Cgl-HS-1 strain of Example 2-1 by electroporation, and through a secondary crossover process, a strain was obtained, in which the wild-type lysC gene on the chromosome was replaced with the mutant lysC(L377K) gene.

The genetic manipulation was confirmed by performing PCR and genome sequencing using a pair of primers of SEQ ID NO: 81 and SEQ ID NO: 82, which are able to amplify the external regions of the homologous recombination upstream and downstream regions where the corresponding gene was inserted, respectively.

The primer sequences used here are as shown in Table 7 below.

**[Table 7]**

| SEQ ID NO. | Sequence name | Sequence (5'→3') |
|---|---|---|
| 75 | lysC promoter Up 1 | TCGAGCTCGGTACCCGACAGGACAAG CACTGGTTG |
| 76 | lysC promoter Up 2 | AGTAGCGCTGGGATGTTTCTCTTTGT GCACCTTTC |
| 77 | lysC Down 1 | GAACATCGAAAAGATTTCCACCTCTGA GAT |
| 78 | lysC Down 2 | CTCTAGAGGATCCCCGTTCACCTCAG AGACGATTA |
| 79 | lysC 1 | CGAAAGGAAACACTCATGGCCCTGGT CGTACAGAA |
| 80 | lysC 2 | GGTGGAAATCTTTTCGATGTTCACGTT GAC |
| 81 | confirm lysC 1 | ACATTCCACCCATTACTGCA |
| 82 | confirm lysC 2 | TCTTCATCGGTTTCGAAGGT |

The transformed strain obtained above was named Cgl-HS-2.

### Example 2-3. Preparation of RhtA mutation-introduced Corynebacterium strain

The pDCM2-PgapA_rhtA, pDCM2-PgapA_rhtA(m1), pDCM2-PgapA_rhtA(m2), pDCM2-PgapA_rhtA(m3), pDCM2-PgapA_rhtA(m4), pDCM2-PgapA_rhtA(m5), pDCM2-PgapA_rhtA(m6), and pDCM2-PgapA_rhtA(m7) vectors prepared in Example 1-4 were transformed into the Cgl-HS-2 strain of Example 2-2 by electroporation, respectively, and through a secondary crossover process, strains were obtained, in which the wild-type rhtA gene or 7 types of the mutant rhtA genes were each inserted on the chromosome. The genetic manipulation was confirmed by performing PCR and genome sequencing using a pair of primers of SEQ ID NO: 83 and SEQ ID NO: 84, which are able to amplify the external regions of the homologous recombination upstream and downstream regions where the corresponding gene was inserted, respectively.

The primer sequences used here are as shown in Table 8 below.

**[Table 8]**

| SEQ ID NO. | Sequence name | Sequence (5'→3') |
|---|---|---|
| 83 | outside F | CACGTGCAGATTCGGTCTCG |
| 84 | outside R | TTCGGTTCTTCAACGTCTGG |

The transformed strains obtained above were named Cgl-HS-3, Cgl-HS-3(m1), Cgl-HS-3(m2), Cgl-HS-3(m3), Cgl-HS-3(m4), Cgl-HS-3(m5), Cgl-HS-3(m6), and Cgl-HS-3(m7), respectively.

### Example 2-4. L-homoserine producing ability of RhtA mutation-introduced Corynebacterium strains

In order to compare the L-homoserine production amounts between Cgl-HS-2 of Example 2-2 and Cgl-HS-3, Cgl-HS-3(m1), Cgl-HS-3(m2), Cgl-HS-3(m3), Cgl-HS-3(m4), Cgl-HS-3(m5), Cgl-HS-3(m6), and Cgl-HS-3(m7) strains of Example 2-3, they were cultured by the following method. In detail, each strain was inoculated into a 250 mL corner-baffled flask comprising 25 mL of a production medium, and cultured at 30°C for 24 hours with shaking at 200 rpm. After completing the culture, the L-homoserine production amounts were measured by HPLC, and the results are shown in Table 9 below.

### <Production medium (pH 7.0)>

Glucose 45 g, (NH₄)₂SO₄ 20 g, MgSO₄·7H₂O 1.2 g, KH₂PO₄ 1.1 g, Biotin 900 µg, Thiamine hydrochloride 4500 µg, Calcium-pantothenic acid 4500 µg, CaCO₃ 30 g (based on 1 liter of distilled water)

**[Table 9]**

| Strain name | L-homoserine production amount (g/L) | L-homoserine yield (*100 g/g, %) |
|---|---|---|
| Cgl-HS-2 | 0.3 | 0.6 |
| Cgl-HS-3 | 0.9 | 1.8 |
| Cgl-HS-3(m1) | 1.9 | 3.6 |
| Cgl-HS-3(m2) | 2.5 | 5.0 |
| Cgl-HS-3(m3) | 3.2 | 6.4 |
| Cgl-HS-3(m4) | 1.7 | 3.4 |
| Cgl-HS-3(m5) | 1.1 | 2.2 |
| Cgl-HS-3(m6) | 1.0 | 2.0 |
| Cgl-HS-3(m7) | 3.0 | 6.0 |

As shown in Table 9, the Cgl-HS-2 strain of Example 2-2 produced 0.3 g/L of L-homoserine, and the Cgl-HS-3 strain, into which the PgapA_rhtA wild-type trait was introduced using the above strain as a parent strain, produced 0.9 g/L of L-homoserine. Further, Cgl-HS-3(m1), Cgl-HS-3(m2), Cgl-HS-3(m3), Cgl-HS-3(m4), Cgl-HS-3(m5), Cgl-HS-3(m6), and Cgl-HS-3(m7), each into which the PgapA_rhtA mutant trait was introduced, produced 1.9 g/L, 2.5 g/L, 3.2 g/L, 1,7 g/L, 1.1 g/L, 1.0 g/L, and 3.0 g/L of L-homoserine, respectively.

The L-homoserine fermentation yield of Cgl-HS-3, into which the PgapA_rhtA wild-type trait was introduced, increased by 1.2%, as compared to that of Cgl-HS-2, whereas the yields of Cgl-HS-3(m1), Cgl-HS-3(m2), Cgl-HS-3(m3), Cgl-HS-3(m4), Cgl-HS-3(m5), Cgl-HS-3(m6), and Cgl-HS-3(m7) strains, into which the PgapA_rhtA mutant trait was introduced, increased by 3%, 4.4%, 5.8%, 2.8%, 1.6%, 1.4%, and 5.4%, respectively, as compared to that of Cgl-HS-2. In particular, the yield increase rates of Cgl-HS-3(m3) and Cgl-HS-3(m7) with respect to Cgl-HS-2 increased by about 4.8 times and 4.5 times, respectively, as compared to the yield increase rate of Cgl-HS-3 with respect to Cgl-HS-2.

### Example 3. rhtA saturated mutagenesis

In Example 1-3 and Table 9, it was confirmed that the Cgl-HS-3(m3) strain comprising the rhtA(V123A) mutation had superior L-homoserine-producing ability, as compared to the strain expressing the wild-type rhtA. Therefore, it was intended to verify the effectiveness of position 123 in improving L-homoserine export by mutating valine, which is the amino acid at position 123 in the amino acid sequence of RhtA, to another amino acid. In order to mutate valine, which is the amino acid at position 123 in the amino acid sequence of RhtA, to 19 types of amino acids other than valine, site-directed mutagenesis was performed by the following method.

In detail, a PCR composition was prepared according to Table 10 below using pDCM2-PgapA_rhtA of Example 1-4 as a template, and PCR was performed. PCR was performed under conditions of denaturation at 95°C for 5 minutes, followed by 30 cycles of denaturation at 95°C for 30 seconds, annealing at 55°C for 1 minute, and polymerization at 68°C for 10 minutes. The mutagenic primer sets in Table 11 below were used when performing PCR.

**[Table 10]**

| .Composition | Content (µL) |
|---|---|
| 10X pfu-X Buffer | 5 |
| 10mM dNTP Mix | 1 |
| pfu-X Polymerase | 1 |
| Mutagenic forward primer(5 pmol) | 2 |
| Mutagenic reverse primer(5 pmol) | 2 |
| pDCM2-PgapA_rhtA(template DNA, 200 ng/µl) | 1 |
| dH₂O | 38 |
| Total | 50 |

**[Table 11]**

| rhtA mutant plasmid | SEQ ID NO. | Sequence name | Sequence (5'→3') |
|---|---|---|---|
| pDCM2-PgapA_rhtA V123A | 85 | pDCM2-PgapA_rhtA V123A F | CGTCGCCCGGTAGATTTCGCCTG GGTTGTGCTGGC |
| | 86 | pDCM2-PgapA_rhtA V123A R | GAAATCTACCGGGCGACGAG |
| pDCM2-PgapA_rhtA V123C | 87 | pDCM2-PgapA_rhtA V123C F | CGTCGCCCGGTAGATTTCTGCTG GGTTGTGCTGGC |
| | 88 | pDCM2-PgapA_rhtA V123C R | GAAATCTACCGGGCGACGAG |
| pDCM2-PgapA_rhtA V123D | 89 | pDCM2-PgapA_rhtA V123D F | CGTCGCCCGGTAGATTTCGACTG GGTTGTGCTGGC |
| | 90 | pDCM2-PgapA_rhtA V123D R | GAAATCTACCGGGCGACGAG |
| pDCM2-PgapA_rhtA V123E | 91 | pDCM2-PgapA_rhtA V123E F | CGTCGCCCGGTAGATTTCGAATG GGTTGTGCTGGC |
| | 92 | pDCM2-PgapA_rhtA V123E R | GAAATCTACCGGGCGACGAG |
| pDCM2-PgapA_rhtA V123F | 93 | pDCM2-PgapA_rhtA V123F F | CGTCGCCCGGTAGATTTCTTCTGG GTTGTGCTGGC |
| | 94 | pDCM2-PgapA_rhtA V123F R | GAAATCTACCGGGCGACGAG |
| pDCM2-PgapA_rhtA V123G | 95 | pDCM2-PgapA_rhtA V123G F | CGTCGCCCGGTAGATTTCGGCTG GGTTGTGCTGGC |
| | 96 | pDCM2-PgapA_rhtA V123G R | GAAATCTACCGGGCGACGAG |
| pDCM2-PgapA_rhtA V123H | 97 | pDCM2-PgapA_rhtA V123H F | CGTCGCCCGGTAGATTTCCACTG GGTTGTGCTGGC |
| | 98 | pDCM2-PgapA_rhtA V123H R | GAAATCTACCGGGCGACGAG |
| pDCM2-PgapA_rhtA V1231 | 99 | pDCM2-PgapA_rhtA V123I F | CGTCGCCCGGTAGATTTCATCTGG GTTGTGCTGGC |
| | 100 | pDCM2-PgapA_rhtA V123I R | GAAATCTACCGGGCGACGAG |
| pDCM2-PgapA_rhtA V123K | 101 | pDCM2-PgapA_rhtA V123K F | CGTCGCCCGGTAGATTTCAAGTG GGTTGTGCTGGC |
| | 102 | pDCM2-PgapA_rhtA V123K R | GAAATCTACCGGGCGACGAG |
| pDCM2-PgapA_rhtA V123L | 103 | pDCM2-PgapA_rhtA V123L F | CGTCGCCCGGTAGATTTCCTCTG GGTTGTGCTGGC |
| | 104 | pDCM2-PgapA_rhtA V123L R | GAAATCTACCGGGCGACGAG |
| pDCM2-PgapA_rhtA | 105 | pDCM2-PgapA_rhtA V123M F | CGTCGCCCGGTAGATTTCATGTG GGTTGTGCTGGC |
| V123M | 106 | pDCM2-PgapA_rhtA V123M R | GAAATCTACCGGGCGACGAG |
| pDCM2-PgapA_rhtA V123N | 107 | pDCM2-PgapA_rhtA V123N F | CGTCGCCCGGTAGATTTCAACTG GGTTGTGCTGGC |
| | 108 | pDCM2-PgapA_rhtA V123N R | GAAATCTACCGGGCGACGAG |
| pDCM2-PgapA_rhtA V123P | 109 | pDCM2-PgapA_rhtA V123P F | CGTCGCCCGGTAGATTTCCCATG GGTTGTGCTGGC |
| | 110 | pDCM2-PgapA_rhtA V123P R | GAAATCTACCGGGCGACGAG |
| pDCM2-PgapA_rhtA V123Q | 111 | pDCM2-PgapA_rhtA V123Q F | CGTCGCCCGGTAGATTTCCAGTG GGTTGTGCTGGC |
| | 112 | pDCM2-PgapA_rhtA V123Q R | GAAATCTACCGGGCGACGAG |
| pDCM2-PgapA_rhtA V123R | 113 | pDCM2-PgapA_rhtA V123R F | CGTCGCCCGGTAGATTTCCGCTG GGTTGTGCTGGC |
| | 114 | pDCM2-PgapA_rhtA V123R R | GAAATCTACCGGGCGACGAG |
| pDCM2-PgapA_rhtA V123S | 115 | pDCM2-PgapA_rhtA V123S F | CGTCGCCCGGTAGATTTCTCCTG GGTTGTGCTGGC |
| | 116 | pDCM2-PgapA_rhtA V123S R | GAAATCTACCGGGCGACGAG |
| pDCM2-PgapA_rhtA V123T | 117 | pDCM2-PgapA_rhtA V123T F | CGTCGCCCGGTAGATTTCACCTG GGTTGTGCTGGC |
| | 118 | pDCM2-PgapA_rhtA V123T R | GAAATCTACCGGGCGACGAG |
| pDCM2-PgapA_rhtA V123W | 119 | pDCM2-PgapA_rhtA V123W F | CGTCGCCCGGTAGATTTCTGGTG GGTTGTGCTGGC |
| | 120 | pDCM2-PgapA_rhtA V123W R | GAAATCTACCGGGCGACGAG |
| pDCM2-PgapA_rhtA V123Y | 121 | pDCM2-PgapA_rhtA V123Y F | CGTCGCCCGGTAGATTTCTACTGG GTTGTGCTGGC |
| | 122 | pDCM2-PgapA_rhtA V123Y R | GAAATCTACCGGGCGACGAG |

1 µL of Dpnl restriction enzyme was added to the gene fragments obtained above, and treated at 37°C for 1 hour, and each gene fragment treated with Dpnl was cloned into a pDCM2 vector using the Gibson assembly method to obtain recombinant mutant plasmids. 3 µL of the recombinant plasmid DNA was transformed into DH5α competent cells to obtain pDCM2-PgapA_rhtA mutant plasmids, and sequencing was performed to confirm that each mutant plasmid comprised rhtA(V123A), rhtA(V123C), rhtA(V123D), rhtA(V123E), rhtA(V123F), rhtA(V123G), rhtA(V123H), rhtA(V123I), rhtA(V123K), rhtA(V123L), rhtA(V123M), rhtA(V123N), rhtA(V123P), rhtA(V123Q), rhtA(V123R), rhtA(V123S), rhtA(V123T), rhtA(V123W), or rhtA(V123Y) mutation, respectively.

The pDCM2-PgapA_rhtA V123A, pDCM2-PgapA_rhtA V123C, pDCM2-PgapA_rhtA V123D, pDCM2-PgapA_rhtA V123E, pDCM2-PgapA_rhtA V123F, pDCM2-PgapA_rhtAV123G, pDCM2-PgapA_rhtAV123H, pDCM2-PgapA_rhtAV123I, pDCM2-PgapA_rhtA V123K, pDCM2-PgapA_rhtA V123L, pDCM2-PgapA_rhtA V123M, pDCM2-PgapA_rhtA V123N, pDCM2-PgapA_rhtA V123P, pDCM2-PgapA_rhtA V123Q, pDCM2-PgapA_rhtA V123R, pDCM2-PgapA_rhtA V123S, pDCM2-PgapA_rhtA V123T, pDCM2-PgapA_rhtA V123W, and pDCM2-PgapA_rhtA V123Y vectors prepared above were each transformed into the Cgl-HS-3 strain using the method of Example 2-3 by electroporation, and through a secondary crossover process, strains in which 19 types of the mutant rhtA genes were each inserted on the chromosome, were obtained, respectively. The genetic manipulation was confirmed by performing PCR and genome sequencing using a pair of primers of SEQ ID NO: 83 and SEQ ID NO: 84, which are able to amplify the external regions of the homologous recombination upstream and downstream regions where the corresponding gene was inserted, respectively.

The transformed strains obtained above were named Cgl-HS-3(V123A), Cgl-HS-3(V123C), Cgl-HS-3(V123D), Cgl-HS-3(V123E), Cgl-HS-3(V123F), Cgl-HS-3(V123G), Cgl-HS-3(V123H), Cgl-HS-3(V123I), Cgl-HS-3(V123K), Cgl-HS-3(V123L), Cgl-HS-3(V123M), Cgl-HS-3(V123N), Cgl-HS-3(V123P), Cgl-HS-3(V123Q), Cgl-HS-3(V123R), Cgl-HS-3(V123S), Cgl-HS-3(V123T), Cgl-HS-3(V123W), and Cgl-HS-3(V123Y), respectively.

### Example 4. L-homoserine-producing ability of RhtA mutation-introduced Corynebacterium strains

In order to compare the L-homoserine production amounts between Cgl-HS-2 of Example 2-2, Cgl-HS-3 of Example 2-3, and the strains prepared in Example 3, they were cultured by the method of Example 2-4. After completing the culture, the L-homoserine production amounts were measured by HPLC, and the results are shown in Table 12 below.

**[Table 12]**

| Strain name | rhtA type | L-homoserine production amount (g/L) | L-homoserine yield (g/g, %) |
|---|---|---|---|
| Cgl-HS-2 | - | 0.3 | 0.6 |
| Cgl-HS-3 | wild-type rhtA | 0.9 | 1.8 |
| Cgl-HS-3(V123A) | rhtA V123A | 3.2 | 6.4 |
| Cgl-HS-3(V123C) | rhtA V123C | 1.0 | 2.0 |
| Cgl-HS-3(V123D) | rhtA V123D | 1.7 | 3.4 |
| Cgl-HS-3(V123F) | rhtA V123F | 1.3 | 2.6 |
| Cgl-HS-3(V123G) | rhtA V123G | 2.5 | 5.0 |
| Cgl-HS-3(V123H) | rhtA V123H | 1.1 | 2.2 |
| Cgl-HS-3(V123I) | rhtA V123I | 1.1 | 2.2 |
| Cgl-HS-3(V123L) | rhtA V123L | 3.5 | 7.0 |
| Cgl-HS-3(V123M) | rhtA V123M | 1.5 | 3.0 |
| Cgl-HS-3(V123N) | rhtA V123N | 1.0 | 2.0 |
| Cgl-HS-3(V123Q) | rhtA V123Q | 1.0 | 2.0 |
| Cgl-HS-3(V123S) | rhtA V123S | 1.6 | 3.2 |
| Cgl-HS-3(V123T) | rhtA V123T | 2.8 | 5.6 |
| Cgl-HS-3(V123W) | rhtA V123W | 1.1 | 2.2 |
| Cgl-HS-3(V123Y) | rhtA V123Y | 1.2 | 2.4 |

As shown in Table 12, the Cgl-HS-3 strains, into which the rhtA(V123A), rhtA(V123C), rhtA(V123D), rhtA(V123F), rhtA(V123G), rhtA(V123H), rhtA(V123I), rhtA(V123L), rhtA(V123M), rhtA(V123N), rhtA(V123Q), rhtA(V123S), rhtA(V123T), rhtA(V123W), or rhtA(V123Y) mutation was introduced, showed increased L-homoserine fermentation yield, as compared to the Cgl-HS-3 strain, into which the PgapA_rhtA wild-type trait was introduced. Accordingly, it was confirmed that the strain expressing RhtA, in which valine, which is the amino acid at position 123 in the amino acid sequence of RhtA, was mutated to alanine, cysteine, aspartic acid, phenylalanine, glycine, histidine, isoleucine, leucine, methionine, asparagine, glutamine, serine, threonine, tryptophan, or tyrosine, had excellent L-homoserine production, as compared to the strain expressing the wild-type RhtA.

### Example 5. Threonine-producing ability of RhtA mutation-introduced Corynebacterium strains

In order to compare the threonine production amounts between Cgl-HS-2 of Example 2-2, Cgl-HS-3 strain of Example 2-3, and Cgl-HS-3 strains of Example 4 comprising the variant in which valine, which is the amino acid at position 123 in the amino acid sequence of RhtA, was mutated to another amino acid, they were cultured by the method of Example 2-4. Among the strains presented in Table 12, Cgl-HS-3(V123A) was used as a representative strain of the Cgl-HS-3 strains comprising the above variant. After completing the culture, the threonine production amounts were measured by HPLC, and the results are shown in Table 13 below.

**[Table 13]**

| Strain name | rhtA type | Threonine production amount (g/L) | Threonine yield (g/g, %) |
|---|---|---|---|
| Cgl-HS-2 | - | 0.2 | 0.4 |
| Cgl-HS-3 | Wild-type rhtA | 1.4 | 2.8 |
| Cgl-HS-3(V123A) | rhtA V123A | 1.9 | 3.8 |

As shown in Table 13, the Cgl-HS-3 strain, into which the V123A mutation was introduced, showed increased threonine fermentation yield, as compared to the Cgl-HS-3 strain, into which the PgapA_rhtA wild-type trait was introduced. Accordingly, it was confirmed that the strain expressing RhtA, in which valine, which is the amino acid at position 123 in the amino acid sequence of RhtA, was mutated to another amino acid, had excellent threonine production, as compared to the strain expressing the wild-type RhtA.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A threonine/homoserine export protein variant, wherein an amino acid corresponding to position 123 in an amino acid sequence of SEQ ID NO: 1 is substituted with a different amino acid.

2. The threonine/homoserine export protein variant of claim 1, wherein the different amino acid is an amino acid selected from the group consisting of alanine, cysteine, aspartic acid, glutamic acid, phenylalanine, glycine, histidine, isoleucine, lysine, leucine, methionine, asparagine, proline, glutamine, arginine, serine, threonine, tryptophan, and tyrosine.

3. The threonine/homoserine export protein variant of claim 2, wherein the different amino acid is an amino acid selected from the group consisting of alanine, cysteine, aspartic acid, phenylalanine, glycine, histidine, isoleucine, leucine, methionine, asparagine, glutamine, serine, threonine, tryptophan, and tyrosine.

4. The threonine/homoserine export protein variant of claim 1, wherein the protein has 80% or more and less than 100% sequence homology to SEQ ID NO: 1.

5. A polynucleotide encoding the threonine/homoserine export protein variant of any one of claims 1 to 3.

6. The polynucleotide of claim 5, wherein the polynucleotide has 80% or more and less than 100% sequence homology to SEQ ID NO: 2.

7. A microorganism comprising any one or more selected from a threonine/homoserine export protein variant, wherein an amino acid corresponding to position 123 in an amino acid sequence of SEQ ID NO: 1 is substituted with a different amino acid; a polynucleotide encoding the variant; or a vector comprising the polynucleotide.

8. The microorganism of claim 7, wherein the microorganism has an increased L-amino acid-producing ability, as compared to a microorganism comprising a wild-type threonine/homoserine export protein having an amino acid sequence of SEQ ID NO: 1 or a polynucleotide encoding the same.

9. The microorganism of claim 7, wherein the L-amino acid is any one or more selected from the group consisting of L-homoserine, O-acetyl-L-homoserine, O-succinyl-L-homoserine, L-methionine, and L-threonine.

10. The microorganism of claim 7, wherein the microorganism is a microorganism of the genus *Corynebacterium.*

11. The microorganism of claim 10, wherein the microorganism of the genus *Corynebacterium* is *Corynebacterium glutamicum.*

12. A method of producing an L-amino acid, the method comprising the step of culturing, in a medium, a microorganism comprising any one or more selected from a threonine/homoserine export protein variant, wherein an amino acid corresponding to position 123 in an amino acid sequence of SEQ ID NO: 1 is substituted with a different amino acid; a polynucleotide encoding the variant; or a vector comprising the polynucleotide.

13. The method of claim 12, wherein the L-amino acid is any one or more selected from the group consisting of L-homoserine, O-acetyl-L-homoserine, O-succinyl-L-homoserine, L-methionine, and L-threonine.

14. Use of a threonine/homoserine export protein variant, wherein an amino acid corresponding to position 123 in an amino acid sequence of SEQ ID NO: 1 is substituted with a different amino acid, in producing an L-amino acid.

15. Use of a microorganism comprising any one or more selected from a threonine/homoserine export protein variant, wherein an amino acid corresponding to position 123 in an amino acid sequence of SEQ ID NO: 1 is substituted with a different amino acid; a polynucleotide encoding the variant; or a vector comprising the polynucleotide, in producing an L-amino acid.
